# EUROPEAN PATENT APPLICATION

(11) **EP 3 208 329 A1**
(43) Date of publication of application: **23.08.2017**
(21) Application number: 15849882.4
(22) Date of filing: 13.10.2015
(51) Int. Cl.: C12N 5/0783, A61K 35/12, A61P 25/00, A61P 31/04, A61P 35/00, A61P 35/02, C12N 5/078

(54) **METHOD FOR PRODUCING GAMMA-DELTA T CELLS, AND PHARMACEUTICAL**

(30) Priority: 14.10.2014 JP 2014209669
(71) Applicant: St. Marianna University School of Medicine, Kawasaki-shi Kanagawa 216-8511 (JP); National University Corporation Hokkaido University, Sapporo-shi, Hokkaido 060-0808 (JP); Medinet Co., Ltd., Yokohama-shi, Kanagawa 222-0033 (JP)
(72) Inventor: YAMANO Yoshihisa, Kawasaki-shi Kanagawa 216-8511 (JP); SEINO Ken-ichiro, Sapporo-shi Hokkaido 060-0808 (JP); MUTO Masato, Tokyo 158-0096 (JP)
(74) Representative: Carpmaels & Ransford LLP
(86) International application number: PCT/JP2015/078911
(87) International publication number: WO 2016/060111

(57) **Abstract**

The purpose of the present invention is to provide a method for producing γδT cells with which it is possible to obtain a cell population including a large amount of yδT cells and including virtually no cells infected by human T-cell leukemia virus (HTLV) even when PBMCs derived from a person infected with HTLV are used as the cell source. A cell population including a large amount of yδT cells and including virtually no HTLV-1-infected cells is obtained by culturing after removing CD4-positive cells that express CD4 (referred to hereinafter as "CD4 + cells") and CD8-positive cells that express CD8 (referred to hereinafter as "CD8 + cells") from the PBMCs that are used as the cell source, or after removing cells that express αβT cell receptors (αβTCR) (referred to hereinafter as "αβT cells"), before expanding γδT cells.

## Description

### TECHNICAL FIELD

The present invention relates to a method for producing γδT cells and a pharmaceutical containing γδT cells.

### BACKGROUND ART

Human T-cell leukemia viruses (hereinafter abbreviated as "HTLV") belong to retroviruses, and 4 types of human T-cell leukemia viruses, type 1 to 4 (hereinafter HTLV-1, 2, 3 and 4), have been identified until now. There are simian T-lymphotropic viruses (= STLV) as related viruses. Among these, it is known that HTLV-1 predominantly infects CD4-positive cells, and HTLV-2 predominantly infects CD8-positive cells (see Non-Patent Literatures 1 and 2). A virus invading a T cell of human, a host, is converted from RNA to cDNA in cytoplasm using released reverse transcriptase, and transmitted to the nucleus, and the cDNA is then integrated into host genome DNA (this state is called provirus). Infected T cells do not always die out, and the virus is transmitted from T cells to T cells, and furthermore virus proliferation is also observed by the proliferation of infected T cells. The infection efficiency of HTLVs by free viral particles is very bad, and thus virus infection needs mainly the intercellular contact of infected cells and uninfected cells.

Currently there are about a million or more people infected with HTLV-1 in Japan, and 20 million or more in the world. Some of infected people develop HTLV-1 associated myelopathy (referred to hereinafter as "HAM") (incidence: about 0.3%). In addition, other infected people develop adult T-cell leukemia (referred to hereinafter as "ATL") (incidence: about 5%). It is further reported that HTLV-1 infection causes HTLV-1 associated uveitis and relates to, for example, lung lesions, lesions of joints, myositis, Sjogren syndrome, chronic renal failure, or dermatitis (Non-Patent Literature 3).

It is known that the above diseases such as HAM and ATL are easily developed when the number of HTLV-1 infected cells in the body increases. Therefore, if the number of HTLV-1 1 infected cells in the body of people infected with HTLV-1 (carriers) can be reduced, it is expected that the onset of HAM and ATL can be prevented. Similarly, if the number of HTLV-1 infected cells in the body of HAM patients and ATL patients can be reduced, it is expected that HAM and ATL can be treated. However, the effective preventive method and therapeutic method for HAM and ATL have not been established until now, and the development of preventive method and therapeutic method as early as possible has been strongly desired.

Meanwhile, immune cell therapy has attracted attention as a therapeutic method for cancer in recent years. The immune cell therapy is a therapeutic method in which immune cells expanded and activated ex vivo of a patient are administered to the patient and the immune cells attack cancer cells. The immune cell therapy has an advantage that side effects are very few compared to three major therapies, conventional surgical treatment, radiation therapy and chemotherapy.

The immune cell therapy has various kinds of therapeutic method, and one of them is γδT cell therapy. γδT cells are cells which play a role of natural immunity, and are known to have cytotoxic activity against cancer cells. γδT cell therapy is a therapeutic method in which γδT cells derived from a patient are expanded and activated ex vivo and the prepared γδT cells are then returned to the body of the patient.

In the γδT cell therapy, γδT cells are expanded and activated using peripheral blood mononuclear cells (referred to hereinafter as "PBMCs") obtained from peripheral blood of a patient as a cell source. γδT cells exist at only about 1 to 5% in peripheral blood, and a method in which a bisphosphonate and interleukin-2 (referred to hereinafter as "IL-2") are added to a culture medium, for example, is suggested as a culture method in which a small amount of peripheral blood is collected from a patient and a sufficient number of yδT cells for treatment are prepared (see e.g. Patent Literature 1). By these methods, γδT cell therapy for cancer patients can be carried out.

When a person infected with HTLV-1 contracts cancer other than ATL or infectious diseases, immune cell therapy is considered in order to treat the cancer or infectious diseases. However, there is a risk that in a conventional method for culturing immune cells, HTLV-1 infection is spread in a culture vessel. When a cell population including HTLV-1 infected cells thus prepared is returned to the patient (the person infected with HTLV-1), there is a risk that the number of HTLV-1 infected cells in the body of the patient increases. Because of this, immune cell therapy such as γδT cell therapy has not been available for a person infected with HTLV-1.

As described above, if the number of HTLV-1 infected cells in the body of a person infected with HTLV-1 can be reduced, it is expected that HAM and ATL can be treated or prevented. As a means for reducing the number of HTLV-1 infected cells, the above-described immune cell therapy is considered. However, immune cell treatment for reducing the number of HTLV-1 infected cells has not been established yet.

Patent Literature 2 discloses a method in which CD4-negative cells obtained by removing CD4-positive cells, a host of HTLV-1, from PBMCs are cultured in the presence of IL-2 and a bisphosphonate (zoledronic acid) for the purpose of currying out γδT cell therapy for a person infected with HTLV-1. Such method succeeds in decreasing the proportion of virus contained in a cell population including γδT cells obtained after culturing as compared to conventional culture methods.

### CITATION LIST

### PATENT LITERATURE

PATENT LITERATURE 1: WO 2006/006720 A
PATENT LITERATURE 2: JP 2012-090574 A

### NON-PATENT LITERATURE

NON-PATENT LITERATURE 1: Doueiri et al. Retrovirology 2012, 9:64
NON-PATENT LITERATURE 2: Ijichi S et al. J. Exp. Med. 1992; 176:293-296
NON-PATENT LITERATURE 3: Instruction Guidance for HTLV-1 Carrier by Study Group of Ministry of Health, Labour and Welfare "Fact Finding Survey and General Measure on HTLV-1 Infection and Related Diseases in Japan"

### SUMMARY OF INVENTION

### TECHNICAL PROBLEM

In a case where peripheral blood of a person infected with HTLV-1 is collected, and lymphocytes and the like are cultured and returned to the body for the purpose of, for example, treating cancer, when the virus is contained in a cell suspension to be administered in an amount larger than the amount of virus contained in peripheral blood at the time of collection, the amount of virus in the body of the patient increases, and there is a possibility that the physical conditions of the patient, for example, are affected. In a cell population obtained by the method disclosed in Patent Literature 2, the proportion of the virus decreases in certain patients and carriers. However, as clinical cases increase, a case where the proportion of the virus does not decrease even when using the above method but increases has been found. This has been thought that infected cells other than CD4-positive cells in PBMCs are grown due to IL-2 stimulation and thus the amount of virus per cell increases. Such case has been detected in samples from both patients and carriers, and it has been unable to predict such patients and carriers from other clinical data. Therefore, such patients and carriers have had to abandon γδT cell immunotherapy since, when blood is collected to obtain PBMCs and the above method is used, many HTLV-1 infected cells are contained in a cell population including γδT cells to be returned to the body.

As described above, immune cell therapy as a treatment for diseases by HTLV-1 virus and cancer is expected for people infected with HTLV-1; however, it has been required to establish a method for culturing γδT cells with cytotoxic activity, which are used for immune cell therapy for patients and carriers in whom a sufficient decrease in virus cannot be observed by PBMCs obtained by the method disclosed in Patent Literature 2.

The present invention is made in view of such points, and an object thereof is to provide a method for producing γδT cells with which it is possible to obtain a cell population including a large amount of yδT cells and including virtually no HTLV-1 infected cells even when PBMCs derived from a person infected with HTLV-1 are used as a cell source as described above.

Another object of the present invention is to provide a pharmaceutical for immunotherapy or immune prophylaxis intended for, for example, cancer or infectious diseases, which contains a cell population including a large amount of yδT cells and including virtually no HTLV-1 infected cells.

Yet another object of the present invention is to provide a pharmaceutical for treating or preventing HAM and ATL related to HTLV-1 infection and other diseases and symptoms.

### SOLUTION TO PROBLEM

The present inventors found that a cell population including a large amount of yδT cells and including virtually no HTLV-1 infected cells was obtained by culturing after removing CD4-positive cells that express a surface antigen CD4 (referred to hereinafter as "CD4⁺ cells") and CD8-positive cells that express a surface antigen CD8 (referred to hereinafter as "CD8⁺ cells") from the PBMCs that are used as a cell source, or after removing cells that express αβT cell receptors (αβTCR) (referred to hereinafter as "αβT cells"), before expanding γδT cells. The present inventors also found that γδT cells obtained by such culture method had cytotoxic activity against HTLV-1 infected cells (including leukemia cells). The present inventors completed the present invention with further investigation based on above knowledge.

That is, the first of the present invention relates to the following method for producing γδT cells.
(1) A method for producing γδT cells and/or NK cells, the method including, a step of preparing peripheral blood mononuclear cells, a step of removing either 1) cells that express a surface marker, either CD4 or CD8, or 2) cells that express a surface marker αβTCR from the peripheral blood mononuclear cells, and a step of culturing a cell population obtained by removing cells that express the surface markers in the presence of a bisphosphonate and interleukin-2.
(2) The method for producing γδT cells and/or NK cells according to (1), wherein the surface markers are CD4 and CD8.
(3) The method for producing γδT cells and/or NK cells according to (1), wherein peripheral blood mononuclear cells are obtained from peripheral blood of a person infected with HTLV-1.
(4) The method for producing γδT cells and/or NK cells according to (1), wherein the concentration of the bisphosphonate in the cell culturing is 0.05 to 100 µM, and the concentration of the interleukin-2 in the cell culturing is 50 to 2000 U/mL.
(5) The method for producing γδT cells and/or NK cells according to (1), wherein the bisphosphonate is pamidronic acid, alendronic acid, zoledronic acid, risedronic acid, ibandronic acid, incadronic acid or a salt thereof, or a hydrate thereof.
(6) A pharmaceutical for treating or preventing cancer or infectious diseases, the pharmaceutical containing γδT cells produced by a method for producing γδT cells and/or NK cells, the method including, a step of preparing peripheral blood mononuclear cells, a step of removing either 1) cells that express a surface marker, either CD4 or CD8, or 2) cells that express a surface marker αβTCR from the peripheral blood mononuclear cells, and a step of culturing peripheral blood mononuclear cells obtained by removing cells that express the surface markers in the presence of a bisphosphonate and interleukin-2.
(7) The pharmaceutical according to (6), wherein the surface markers are CD4 and CD8.
(8) The pharmaceutical according to (6), wherein the peripheral blood mononuclear cells are obtained from peripheral blood of a person infected with HTLV-1.
(9) The pharmaceutical according to (8), which is a pharmaceutical for treating or preventing HTLV-1 associated myelopathy or adult T-cell leukemia.

### ADVANTAGEOUS EFFECT OF INVENTION

According to the production method of the present invention, a cell population including a large amount of yδT cells and including virtually no HTLV-1 infected cells can be obtained even when PBMCs derived from a person infected with HTLV-1 are used. The obtained γδT cells have cytotoxic activity against cells of cancer and infectious diseases, and are also expected to have cytotoxic activity against HTLV-1 infected cells. Therefore, according to the production method of the present invention, a cell population including a large amount of yδT cells and including virtually no HTLV-1 infected cells can be obtained from a blood sample derived from a person infected with HTLV-1. Using γδT cells obtained by the production method of the present invention, a pharmaceutical for treating and preventing cancer, infectious diseases or a HTLV-1 related disease caused by HTLV-1 infected cells (e.g. HTLV-1 associated myelopathy or adult T-cell leukemia) can be produced.

### BRIEF DESCRIPTION OF DRAWINGS

FIG.1 is a graph showing the amount of provirus contained in PBMCs derived from a person infected with HTLV-1 before culturing and the amount of provirus contained in a cell population after culturing.
FIG. 2 is a graph showing the expansion rate of yδT cells when PBMCs derived from a person infected with HTLV-1 are cultured by a conventional method, a CD4⁺ removal method, the method of the present invention (CD4⁺CD8⁺ removal method).
FIG. 3 is a graph showing, when PBMCs derived from a person infected with HTLV-1 are cultured by the CD4⁺ removal method, the amount of provirus contained in a cell population before and after culturing.
FIG. 4 is a graph showing, when PBMCs derived from a person infected with HTLV-1 are cultured by the method of the present invention (CD4⁺CD8⁺ removal method), the amount of provirus contained in a cell population before and after culturing.
FIG. 5 is a graph showing, when PBMC derived from a person infected with HTLV-1 are cultured by the conventional method, the CD4⁺ removal method and the method of the present invention (CD4⁺CD8⁺ removal method), the amount of provirus contained in a cell population before and after culturing.
FIG. 6 is a graph showing the rate of virus after culturing PBMCs derived from a person infected with HTLV-1 by the conventional method, the CD4⁺ removal method and the method of the present invention (CD4⁺CD8⁺ removal method). The rate of virus was calculated using the amount of provirus contained at the time of collection as 1.
FIG. 7 is a graph showing the rate of virus after culturing PBMCs derived from a person infected with HTLV-1 by the CD4⁺ removal method and the method of the present invention (CD4⁺CD8⁺ removal method). The rate of virus was calculated using the amount of provirus contained at the time of collection as 1.
FIG. 8 is a graph showing, when PBMC derived from a person infected with HTLV-1 are cultured by the conventional method, the CD4⁺ removal method and the method of the present invention (αβT cell removal method), the amount of provirus contained in a cell population before and after culturing.
FIG. 9 is a graph showing the rate of virus after culturing PBMCs derived from a person infected with HTLV-1 by the CD4⁺ removal method and the method of the present invention (αβT cell removal method). The rate of virus was calculated using the amount of provirus contained at the time of collection as 1.
FIG. 10 is a graph showing the cytotoxic activity of yδT cells, obtained by culturing PBMCs derived from an ATL patient by the method of the present invention (CD4⁺CD8⁺ removal method), against a cell line derived from an ATL patient.
FIG. 11 is a graph showing the cytotoxic activity of yδT cells, obtained by culturing PBMCs derived from a HAM patient by the method of the present invention (CD4⁺CD8⁺ removal method), against a cell line derived from an ATL patient.
FIG. 12 is a graph showing the cytotoxic activity of yδT cells, obtained by culturing PBMCs derived from a HAM patient by the method of the present invention (CD4⁺CD8⁺ removal method), against Daudi cell line in the presence (Rituximab+) or absence (Rituximab-) of Rituximab.
FIG. 13 is a chart showing the analysis results of the CD16 expression of yδT cells, obtained by culturing PBMCs derived from a HAM patient by the method of the present invention (CD4⁺CD8⁺ removal method).

### DESCRIPTION OF EMBODIMENTS

### <1. Method for Producing γδT Cells>

The method for producing γδT cells of the present invention includes 1) a first step of preparing PBMCs, 2) a second step of removing cells that express a predetermined surface marker from the PBMCs, and 3) a third step of culturing residual PBMCs in the presence of a bisphosphonate and IL-2.

The method for producing γδT cells of the present invention has a feature of removing cells that express a predetermined surface marker from PBMCs in the second step. Each step will now be described.

In the first step, PBMCs that are used as a cell source for γδT cells are prepared. Herein, "peripheral blood mononuclear cells (PBMCs)" mean a cell population including lymphocytes (natural killer cells, natural killer T cells, αβT cells, γδT cells, etc.), monocytes and the like separated from peripheral blood. The method for preparing PBMCs is not particularly limited. For example, PBMCs can be obtained by density-gradient centrifugation of peripheral blood obtained by blood collection. The amount of blood collected at a time can be appropriately set depending on a patient who receives γδT cell therapy and is for example about 45 to 75 mL.

When it is required to secure a large amount of cells, PBMCs can be directly obtained by collecting a mononuclear cell component using an apheresis device.

In the second step, cells that express a surface marker, either CD4 or CD8, or cells that express a surface marker αβTCR are removed from PBMCs prepared in the first step.

CD4, CD8 and αβTCR are surface markers which are known to express in target cells of HTLV-1. Therefore, almost all of HTLV-1 infected cells in PBMCs can be removed by removing cells that express these surface markers even if HTLV-1 infected cells are contained in PBMCs (see examples).

The method for removing cells that express the above surface markers from PBMCs is not particularly limited, and can be appropriately selected from known methods. Examples of methods for removing cells that express the above surface markers from PBMCs include magnetic cell sorting, flow cytometry and the like.

In the third step, PBMCs after removing cells that express a predetermined surface marker in the second step are suspended in a culture fluid (medium), and a bisphosphonate and IL-2 are added to the obtained cell suspension to culture γδT cells. PBMCs are cultured in the presence of a bisphosphonate and IL-2 to selectively grow and activate γδT cells, and a cell population including activated γδT cells at a high purity can be prepared (see Patent Literature 1).

The type of culture medium in which PBMCs are suspended is not particularly limited as long as γδT cells can be expanded. Examples of such culture medium include AIM-V medium, RPMI-1640 medium, Dulbecco's Modified Eagle's Medium (DMEM), and Iscove medium. To these culture media, a serum can be added as needed. Examples of serum to be added include fetal bovine serum (FCS), AB serum and autologous plasma.

In the present invention, a bisphosphonate is not particularly limited, and means a compound having a P-C-P skeleton. Examples of bisphosphonates used in the present invention include a compound represented by the following general formula [Chemical Formula 1], salts thereof and hydrates thereof.

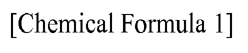

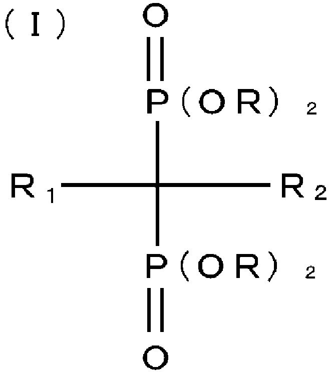

In the above [Chemical Formula 1], R is a hydrogen atom or a lower alkyl group, R₁ and R₂ are each independently selected from the group consisting of a hydrogen atom, a halogen atom, a hydroxyl group, an amino group, a thiol group, an aryl group which may be substituted, an alkyl group which may be substituted, a lower alkylamino group, an aralkyl group, a cycloalkyl group and a heterocyclic group, and R₁ and R₂ may form a part of an identical cyclic structure.

The substituents in the above R₁ and R₂ are selected from the group consisting of, for example, a halogen atom, a lower alkyl group, a hydroxyl group, a thiol group, an amino group, an alkoxy group, an aryl group, an arylthio group, an aryloxy group, an alkylthio group, a cycloalkyl group, a heterocyclic group and the like.

In the present description, examples of halogen atoms are a fluoro atom, a chloro atom, a bromine atom and the like; examples of alkyl groups are straight or branched C₁-C₃₀ alkyl groups such as methyl, ethyl, propyl, isopropyl, butyl, pentyl, heptyl, octyl, and pentadecanyl and the like; examples of lower alkyl groups are straight or branched C₁-C₁₀ alkyl groups and the like; examples of aryl groups are phenyl, naphthyl and the like; examples of aralkyl groups are aryl-lower alkyl groups and the like; examples of cycloalkyl groups are C₁-C₁₀ cycloalkyl groups such as cyclooctyl and adamantly and the like; heterocyclic groups are pyridyl, furyl, pyrrolidinyl, imidazolyl, quinolyl, isoquinolyl and the like.

In the present invention, bisphosphonates are preferably pharmaceutically acceptable ones, and include those which have a bone resorption inhibitory behavior and are generally used as an osteoporosis drug. Examples thereof include pamidronic acid, alendronic acid, zoledronic acid, risedronic acid, ibandronic acid, incadronic acid and salts thereof, and hydrates thereof. These are aminobisphosphonates having a nitrogen atom, and pamidronic acid, alendronic acid, zoledronic acid and salts thereof, and hydrates thereof are particularly preferred. Examples of commercially available bisphosphonate-based bone metabolism improving agents include pamidronate disodium pentahydrate (AREDIA (registered trademark); Novartis Pharma K.K.), zoledronic acid hydrate (ZOMETA (registered trademark); Novartis Pharma K.K.) and the like.

The concentration of bisphosphonate added when culturing PBMCs is preferably 0.05 to 100 µM, and more preferably 0.1 to 30 µM. More specifically, when pamidronic acid or a salt thereof, or a hydrate thereof, or alendronic acid or a salt thereof, or a hydrate thereof is added as a bisphosphonate, the concentration of bisphosphonate is preferably 1 to 30 µM. In addition, when zoledronic acid or a salt thereof, or hydrate thereof is added as a bisphosphonate, the concentration of bisphosphonate is preferably 0.1 to 10 µM.

The concentration of IL-2 added when culturing PBMCs is preferably 50 to 2000 U/mL, and more preferably 400 to 1000 U/mL.

The culture conditions of PBMCs are not particularly limited as long as γδT cells can be expanded and activated. In general, it is only needed that the cells should be cultured at 34 to 38°C (preferably 37°C) in the presence of 2 to 10% (preferably 5%) CO₂ for about 7 to 14 days. At this time, a culture medium is appropriately added depending on the number of cells to be cultured. Furthermore, IL-2 is appropriately added so that the concentration will be 50 to 2000 U/mL and more preferably 400 to 1000 U/mL depending on an increase in the amount of culture medium.

By the above procedure, a cell population including a large amount of yδT cells can be obtained. The obtained cell population can be used as cells which are administered to patients in γδT cell therapy as described below.

In the method for producing γδT cells of the present invention, HTLV-1 infected cells are removed in the second step, and thus even a case where HTLV-1 infected cells are contained in PBMCs prepared in the first step is not particularly a problem. Therefore, the method for producing γδT cells of the present invention can be used for PBMCs derived from a person infected with HTLV-1. By using the method for producing γδT cells of the present invention, the amount of virus after culturing is reduced in PBMCs derived from peripheral blood of a person infected with HTLV-1, and furthermore a cell population including a large amount of yδT cells can be produced.

Furthermore, in addition to γδT cells, immune cells with cytotoxic activity such as natural killer cells (NK cells) can be also stimulated and expanded individually or together with for example γδT cells after removing HTLV-1 infected cells in the above second step. NK cells do not express T cell specific markers (TCR, CD3, CD4 and CD8) and B cell specific markers (Ig, CD 19 and CD20) and are expanded by IL-2 stimulation. Therefore, the cells can be stimulated and expanded simultaneously with γδT cells without influence of cell removal by microbeads according to the invention of the present application. Therefore, γδT cells according to the invention of the present application can contain NK cells. When isolating only NK cells, the two can be separated and purified by a known method after removing CD4⁺ cells and CD8⁺ cells or removing αβT cells. Using a γδT cell specific marker γδTCR or the like, for example, NK cells can be isolated by removing cells that express γδTCR by magnetic cell sorting, flow cytometry or the like.

### <2. Pharmaceutical Containing γδT Cells>

The pharmaceutical of the present invention is a pharmaceutical for treating and preventing, for example, cancer or infectious diseases, which contains γδT cells produced by the above-described production method of the present invention.

The pharmaceutical of the present invention is, for example, an injection (a cell suspension) obtained by suspending γδT cells produced by the production method of the present invention in a liquid available as a pharmaceutical product (e.g. a physiological salt solution). This injection can be administered, for example, by intravenous, intradermal or subcutaneous injection, directly injected to lesions, or systemically administered as a drip.

The pharmaceutical of the present invention contains γδT cells produced by the production method of the present invention as an essential component, and can contain other components as optional components. For example, when the pharmaceutical of the present invention is used as a therapeutic or preventive agent for cancer, cytokines such as IL-2 and IL-12 can be added. In addition, when the pharmaceutical of the present invention is used as a therapeutic or preventive agent for virus infectious diseases, interferon, for example, can be added. Furthermore, the pharmaceutical of the present invention can contain other cells with cytotoxic activity such as NK cells.

The number of yδT cells contained in the pharmaceutical of the present invention can be appropriately set depending on, for example, an administration method, types of disease, and symptoms of patients, and can be usually set to 10⁸ to 10¹² cells/person (preferably 10⁹ cells/person).

The method for producing the pharmaceutical of the present invention is not particularly limited. The pharmaceutical of the present invention can be produced, for example, by 1) collecting γδT cells obtained by the method for producing γδT cells of the present invention, for example, by centrifugation; 2) washing the collected γδT cells with a washing solution (e.g. a physiological salt solution, PBS, etc.); 3) collecting the washed γδT cells, for example, by centrifugation; and 4) suspending the collected γδT cells in a liquid available as a pharmaceutical product (e.g. a physiological salt solution).

As described above, the production method of the present invention can reduce the amount of virus originally contained in PBMCs derived from peripheral blood of a person infected with HTLV-1 and produce a large amount of yδT cells. Therefore, there is less risk that, even when the pharmaceutical containing γδT cells produced by the production method of the present invention is administered to a person infected with HTLV-1, the number of HTLV-1 infected cells originally existing in the body of the infected person increases. Therefore, the pharmaceutical of the present invention can be used as a pharmaceutical for γδT cell therapy for a person infected with HTLV-1.

As shown in examples described below, γδT cells have cytotoxic activity against HTLV-1 infected cells (including leukemia cells of an ATL patient). This is found for the first time by the present inventors. As described above, if the number of HTLV-1 infected cells in the body of a person infected with HTLV-1 can be reduced, it is expected that HAM and ATL can be treated and prevented. Therefore, the pharmaceutical of the present invention can be used as a pharmaceutical for treating and preventing HAM and ATL.

The present invention will now be described in detail with reference to examples thereof. It should be noted however that the present invention is not limited to these examples. Examples

### <1. Culturing of yδT Cells by Conventional Method>

First, a case of culturing γδT cells by a conventional method (a culture method without removing specific cells from PBMCs) is shown as Comparative Example.

### (1) Culturing of yδT Cells

Using heparin as an anticoagulant, flesh blood was collected from a person infected with HTLV-1. The obtained blood was separated into a blood cell component and a blood plasma component by centrifugation. The blood plasma component was inactivated at 56°C for 60 minutes and then frozen at -80°C once. The frozen blood plasma was thawed again and then centrifuged to obtain a supernatant. The obtained supernatant was used as autologous plasma at the time of cell culture. The blood cell component, meanwhile, was diluted with PBS(-) and PBMCs were then separated using a Lymphocyte Separation Media (LSM; Cosmo Bio Co., Ltd.). The separated PBMCs were washed with PBS(-).

The washed PBMCs were suspended in a lymphocyte culture medium (ALyS203; Cell Science & Technology Institute, Inc.) to prepare a cell suspension. The obtained cell suspension was added to a 24 well microplate at 2 x 10⁶ PBMCs/well. Next, IL-2 (Immunotec), zoledronic acid hydrate (ZOMETA (registered trademark); Novartis Pharma K.K.) and autologous plasma were added to each well. The final concentrations of the components are IL-2: 1000 U/mL, zoledronic acid hydrate: 5 µM, and autologous plasma: 10%.

The microplate was transferred to a CO₂ incubator (temperature: 37°C, CO₂ concentration: 5%, humidity: supersaturation), and PBMCs were cultured for 14 days. Every time when the cells became confluent during the period of culturing, an IL-2 added medium was added. In addition, autologous plasma was appropriately added so that the concentration of blood plasma in the medium was not below 1%.

### (2) Flow Cytometry

The proportion of yδT cells contained in cells before culturing (day 0) and after culturing (day 14) was measured using flow cytometry. In this experiment, CD45⁺CD3⁺TCR Vγ9⁺ cells were decided as γδT cells.

As a flow cytometer, FC500 (Beckman Coulter K.K.) was used, and as an analysis software, CXP (Beckman Coulter K.K.) was used. ECD-labeled anti-human CD45 antibody (Beckman Coulter K.K.) was used as an anti-CD45 antibody, and PC5-labeled anti-human CD3 antibody (Beckman Coulter K.K.) was used as an anti-CD3 antibody, and FITC-labeled anti-human TCR Vγ9 antibody (Beckman Coulter K.K.) was used as an anti-TCR Vγ9 antibody.

### (3) Measurement of Amount of HTLV-1 Provirus

The amount of HTLV-1 provirus per 100 cells contained in cells before culturing (day 0) and cells after culturing (day 14) was measured by Real-Time PCR.

A cell population to be measured (cells of day 0 or day 14) was suspended in a lysis buffer (50 mM Tris-HCl (pH 8.0), 20 mM EDTA, 0.1 M NaCl, and 1% SDS). To the obtained cell suspension, proteinase K (Wako Pure Chemical Industries, Ltd.) was added so that the final concentration was 150 µg/mL, and the obtained mixture was shaken at 55°C overnight, and genome DNA was then extracted using phenol-chloroform. Using TaqMan Probe-Primer set for the pX region of HTLV-1 and human β-actin (SEQ ID NOs: 1 to 6), Real-Time PCR was carried out. Using a standard sample, a calibration curve was made to calculate the amount of the pX region of HTLV-1 and human β-actin, and using these values, the amount of HTLV-1 provirus per 100 cells was calculated.

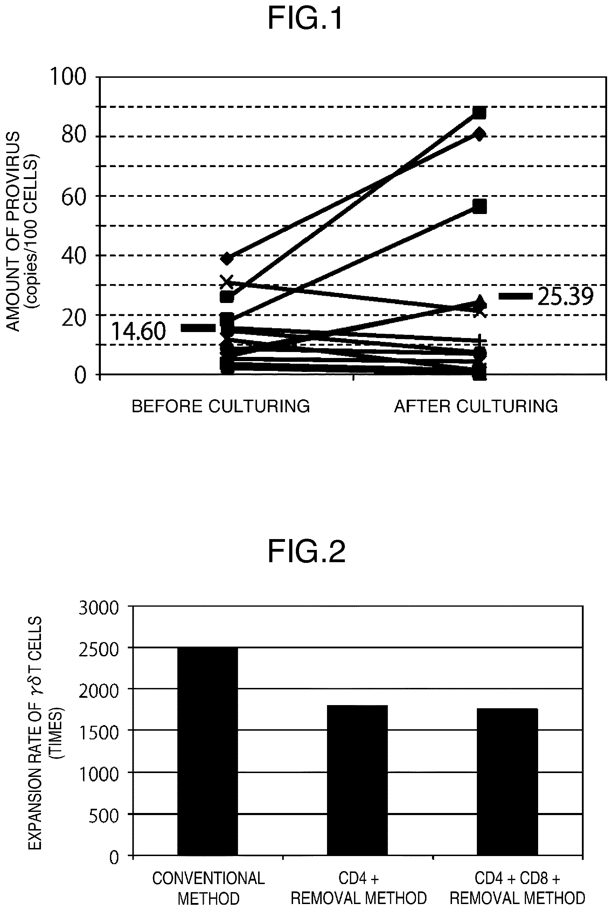

FIG.1 is a graph showing the amount of HTLV-1 provirus per 100 cells contained in a cell population before culturing (day 0) and after culturing (day 14) (n = 13). As shown in this graph, the average amount of virus contained in a cell population before culturing (day 0) was 14.60 copies/100 cells, while the average amount of virus contained in a cell population after culturing (day 14) was 25.39 copies/100 cells.

### <2. Culturing of yδT Cells by Method of Invention>

Next, a case of culturing γδT cells by the method of the present invention (a method in which CD4⁺ cells and CD8⁺ cells are removed from PBMCs) is shown as an example.

### (1) Culturing of yδT Cells

Autologous plasma and PBMCs were obtained from flesh blood of a person infected with HTLV-1 in the same procedure as in Comparative Example above. Next, a cell population obtained by removing CD4⁺ cells from the obtained PBMCs by magnetic cell sorting and a cell population obtained by removing CD4⁺ cells and CD8⁺ cells were prepared. Specifically, PBMCs were allowed to react with anti-CD4 microbeads (Miltenyi Biotec K.K.) and/or anti-CD8 microbeads (Miltenyi Biotec K.K.), and the reactant was then applied to a MACS separation column (Miltenyi Biotec K.K.) to provide a cell population obtained by removing CD4⁺ cells or both CD4⁺ cells and CD8⁺ cells from PBMCs.

The cell population obtained by removing CD4⁺ cells, and the cell population obtained by removing CD4⁺ cells and CD8⁺ cells were cultured for 14 days in the same procedure as in Comparative Example above. The number and proportion of yδT cells contained in PBMCs vary depending on subjects, and thus the increase rates of yδT cells before and after culturing were compared. The results are shown in FIG. 2. It was verified that the increase rates of yδT cells were not different in the method in which CD4⁺ cells are removed and the method in which CD4⁺ cells and CD8⁺ cells are removed.

### (2) Measurement of Amount of HTLV-1 Provirus

The amount of HTLV-1 provirus per 100 cells (infection rate) was measured before culturing (day 0) and after culturing (day 14) of the cell population obtained by removing CD4⁺ cells and the cell population obtained by removing CD4⁺ cells and CD8⁺ cells in the same procedure as in Comparative Example above.

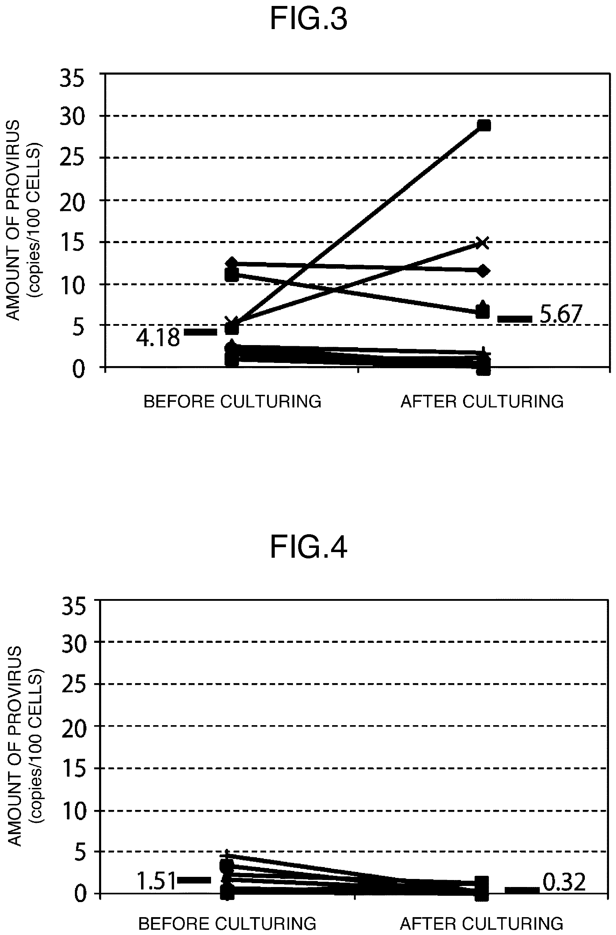

FIG. 3 is a graph showing the amount of HTLV-1 provirus per 100 cells in a cell population before culturing (day 0) and after culturing (day 14) (n = 13). When only CD4⁺ cells were removed, the amount of provirus after culturing could be reduced as compared to that before culturing. In some donors, however, there is a case where the amount of provirus increases, and when calculating the average value of the proportion of provirus, the average value of the proportion of virus before culturing (day 0) was 4.18 copies/100 cells, while the average value of the proportion of provirus after culturing (day 14) was 5.67 copies/100 cells.

FIG. 4 is a graph showing the amount of provirus when culturing was carried out after removing both CD4⁺ cells and CD8⁺ cells (n = 11). In all the donor cells which received a culture test, the amount of virus decreased. When the average value of the proportion of virus was calculated, the average value of the proportion of virus before culturing (day 0) was 1.51 copies/100 cells, while the average value of the proportion of virus after culturing (day 14) was 0.32 copies/100 cells. The proportion of virus after culturing could be reduced as compared to that before culturing.

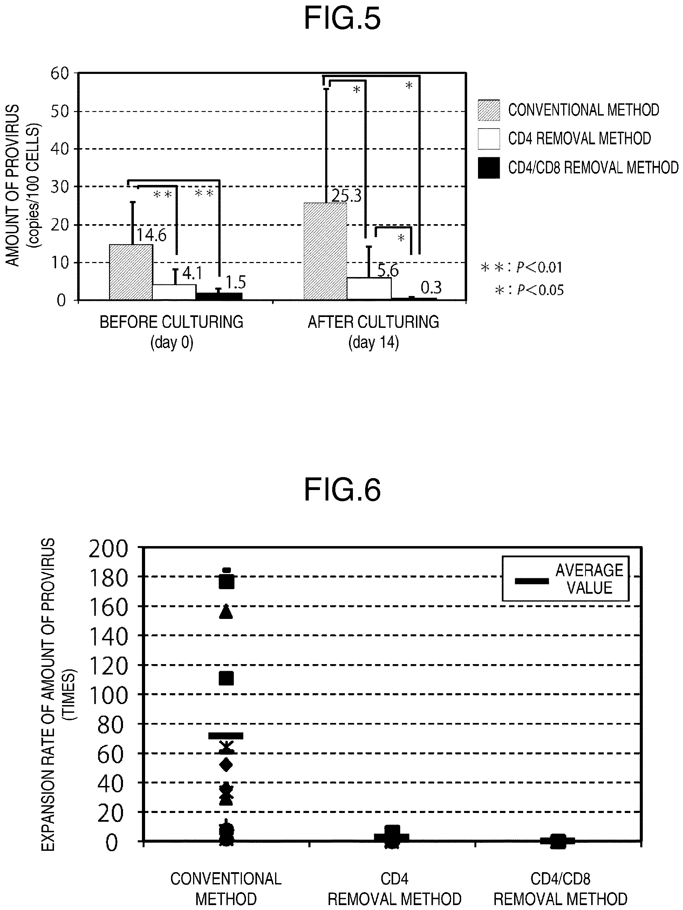

The prior data are summarized and shown in FIG. 5. It was verified that the proportion of provirus contained in a cell population after culturing decreased by the method in which CD4⁺ cells are removed or the method in which both CD4⁺ cells and CD8⁺ cells are removed.

Based on the prior results, when the amount of provirus contained at the time of blood collection was considered as 1, the increase rate of virus contained in a cell population after culturing (day 14) was calculated.

The results are shown in FIG. 6. The increase rate of virus was 71.4 times in the conventional culture method for γδT cells, 3.1 times in the method in which only CD4⁺ cells are removed, and 0.1 times in the method in which CD4⁺ cells and CD8⁺ cells are removed. It was revealed that in the method in which only CD4⁺ cells are removed, the proportion of virus decreased, while the amount of virus increased as compared to that at the time of blood collection. Meanwhile, it was revealed that in the method in which CD4⁺ cells and CD8⁺ cells are removed, not only the proportion of virus but also the amount of virus decreased as compared to those at the time of blood collection.

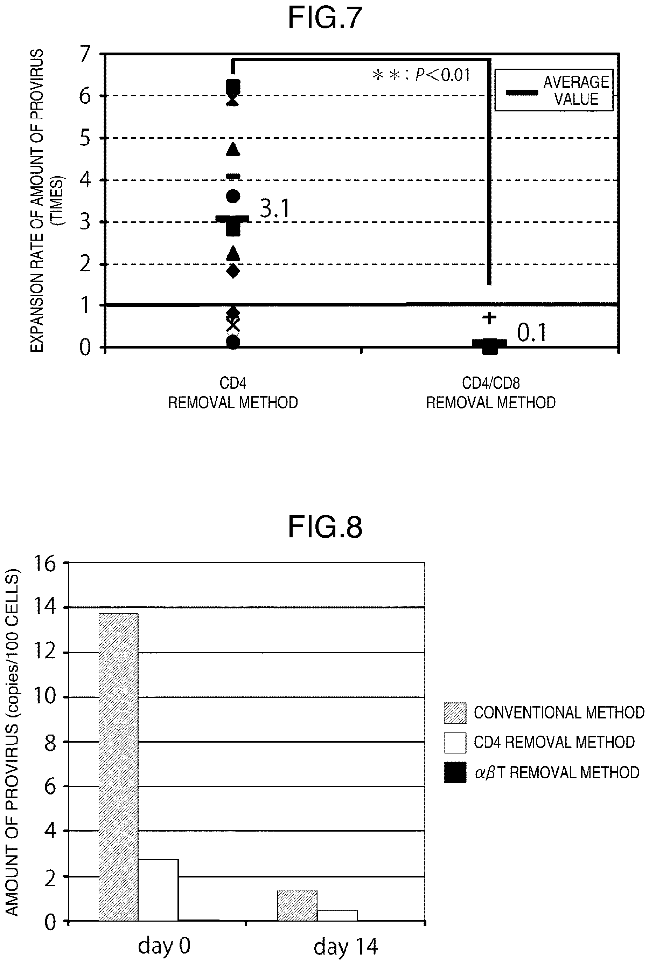

FIG. 7 is a graph showing the data of the method in which only CD4⁺ cells are removed and the method in which CD4⁺ cells and CD8⁺ cells are removed extracted from the data in FIG. 6. When the target increase rate of virus was considered as 1 or less, the number of cases achieving the target was 4 of 13 cases in the method in which only CD4⁺ cells are removed, while all the 11 cases achieved the target value in the method in which CD4⁺ cells and CD8⁺ cells are removed.

Next, a case of culturing γδT cells by the method of the present invention (a method in which αβT cells are removed from PBMCs) is shown as an example.

### (1) Culturing of yδT Cells

Autologous plasma and PBMCs were obtained from flesh blood of a person infected with HTLV-1 in the same procedure as in Comparative Example above. Next, a cell population obtained by removing CD4⁺ cells from the obtained PBMCs by magnetic cell sorting and a cell population obtained by removing αβT cells were prepared. Specifically, PBMCs were allowed to react with anti-CD4 microbeads (Miltenyi Biotec K.K.) or PE-labeled anti-αβT antibody (Miltenyi Biotec K.K.) and anti-PE microbeads (Miltenyi Biotec K.K.), and the reactant was then applied to a MACS separation column (Miltenyi Biotec K.K.) to provide a cell population obtained by removing CD4⁺ cells or αβT cells from PBMCs.

### (2) Measurement of Amount of HTLV-1 Provirus

The amount of HTLV-1 provirus per 100 cells was measured before culturing (day 0) and after culturing (day 14) of the cell population obtained by removing CD4⁺ cells and the cell population obtained by removing αβT cells in the same procedure as in Comparative Example above.

FIG. 8 is a graph showing the amount of HTLV-1 provirus per 100 cells in a cell population before culturing (day 0) and after culturing (day 14) (n = 3). It was verified that the proportion of virus contained in a cell population after culturing decreased by the method in which CD4⁺ cells are removed or the method in which both CD4⁺ cells and CD8⁺ cells are removed as compared to that in the conventional method.

Based on the above results, when the amount of virus per cell contained at the time of blood collection was considered as 1, the increase rate of virus contained in a cell population after culturing (day 14) was calculated.

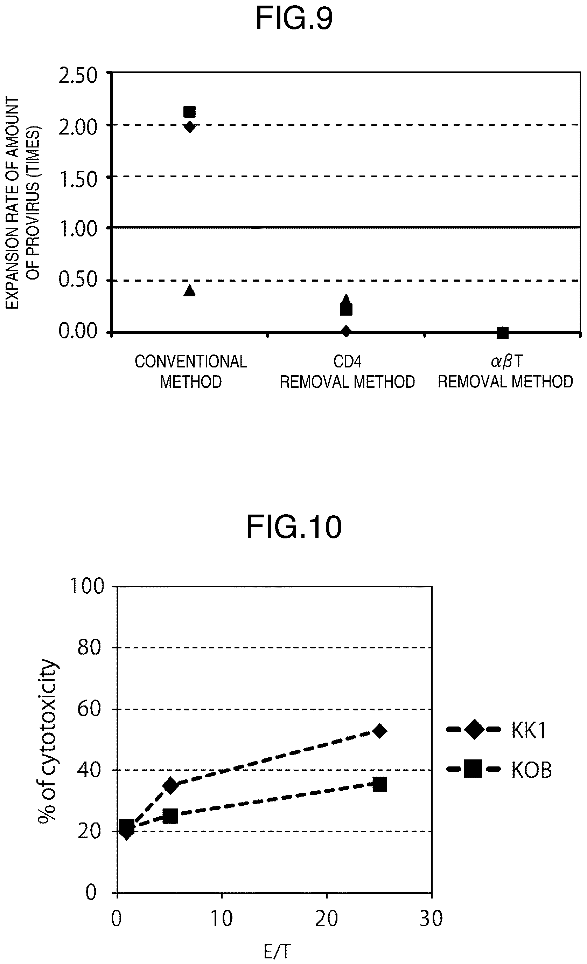

The results are shown in FIG. 9. When the target increase rate of virus was considered as 1, the target value could not be achieved in two cases in the conventional culture method for γδT cells. It was revealed that, in the methods in which only CD4⁺ cells are removed and which αβT cells are removed, the target value was achieved in all the three cases, and the virus was reduced to not more than the detection limit in the method in which αβT cells are removed.

From this result, it was revealed that, even when PBMCs derived from a person infected with HTLV-1 are used as a cell source, the growth of HTLV-1 infected cells could be suppressed by culturing after removing CD4⁺ cells, both CD4⁺ cells and CD8⁺ cells, or αβT cells. It was shown that particularly the method of the present invention (a method in which CD4⁺ cells and CD8⁺ cells are removed, or a method in which αβT cells are removed) was a stable method compared to the method in which CD4⁺ cells are removed as a culture method which reduces the amount of virus originally contained in PBMCs derived from peripheral blood of a person infected with HTLV-1, and which obtains a large amount of yδT cells without affecting the growth ability of yδT cells. A pharmaceutical containing γδT cells produced by the production method of the present invention can be used for γδT cell therapy for a person infected with HTLV-1 since, even when administered to a person infected with HTLV-1, there is less risk that the amount of HTLV-1 infected cells originally existing in the body of the infected person increases.

### (3) Cytotoxic Activity of yδT Cells

The cytotoxic activity of yδT cells obtained by the culture method of the present invention was confirmed.

Blood was collected from an ATL patient or a HAM patient, peripheral blood mononuclear cells were separated, and γδT cells were obtained by the method of the present invention.

The obtained γδT cells (Effector cell) were mixed with a cell line derived from an ATL patient (KK1, KOB: Target cell) so that E/T (Effector cell/Target cell) ratio was 1, 5 or 25, and the obtained mixture was co-cultured for 2 hours. Using Terascan (Minerva Tech K.K.), cytotoxic activity was measured after 2 hours.

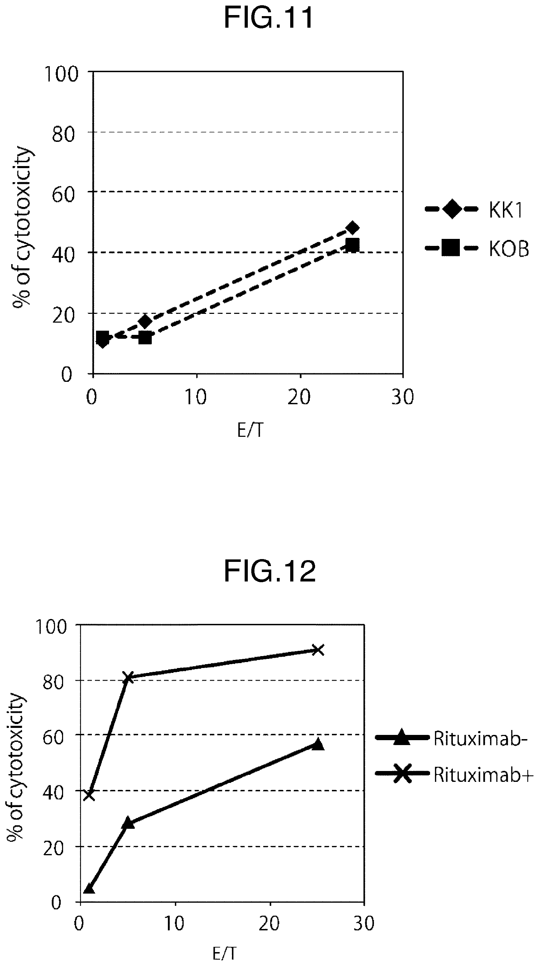

The results are shown in FIGS. 10 and 11. The cytotoxic activity of yδT cells cultured from PBMCs derived from an ATL patient was shown in FIG. 10. It was revealed that γδT cells derived from an ATL patient showed cytotoxic activity against KK1 and KOB.

The cytotoxic activity of yδT cells cultured from PBMCs derived from a HAM patient was shown in FIG. 11. It was revealed that γδT cells derived from a HAM patient showed cytotoxic activity against KK1 and KOB.

The following experiment was carried out to confirm if γδT cells obtained by the method of the present invention have antibody-dependent cellular cytotoxicity (ADCC).

Blood was collected from a HAM patient, peripheral blood mononuclear cells were separated, and γδT cells were obtained by the method of the present invention.

The obtained γδT cells were mixed with Daudi cell line derived from malignant lymphoma (CD20-positive human lymphoma cell line) so that E/T ratio was 1, 5 or 25, and Rituximab (human murine chimeric anti-human CD20 antibody) (Rituxan (registered trademark), Chugai Pharmaceutical Co., Ltd.) was added thereto and the obtained mixture was co-cultured for 2 hours. Using Terascan (Minerva Tech K.K.), cytotoxic activity was measured after 2 hours.

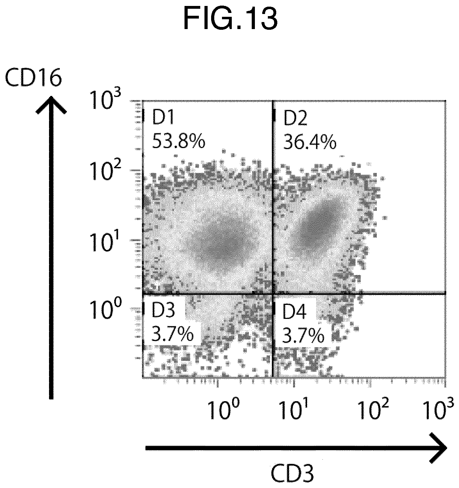

The results are shown in FIG. 12. When Rituximab was added, γδT cells cultured from PBMCs derived from a HAM patient showed high cytotoxic activity against Daudi cell line as compared to when Rituximab was not added. As shown in FIG. 13, it was also verified that CD16 expressed in such γδT cells. From these results, it was revealed that similar to common γδT cells, γδT cells obtained by the present invention also had ADCC.

By the present example, when administered to the body of a patient, γδT cells obtained by the culture method of the present invention are expected to kill HTLV-1 infected cells and cancer cells existing in the body of the patient.

### INDUSTRIAL APPLICABILITY

The production method of the present invention is useful as a method for producing γδT cells, for example, when doing γδT cell therapy for a person infected with HTLV-1. The pharmaceutical of the present invention is useful as a pharmaceutical containing γδT cells which aims for the treatment of cancer, infectious diseases or HTLV-1 related diseases, for example, for a person infected with HTLV-1, a HAM patient and an ATL patient. Furthermore, using antibody-dependent cellular cytotoxicity, the pharmaceutical can be used in combination with an existing antibody drug. It is expected that therapeutic effects are improved by combining the pharmaceutical, for example, with Rituximab when a person infected with HTLV-1 contracts CD20-positive B cell non-Hodgkin lymphoma, Trastuzumab when the person contracts Her2 positive cancer, and Cetuximab when the person contracts metastatic colorectal cancer. Furthermore, it is expected that therapeutic effects are improved by combining the pharmaceutical with Mogamulizumab for a CCR4 positive ATL patient.

## Claims

1. A method for producing γδT cells and/or NK cells, the method comprising,
a step of preparing peripheral blood mononuclear cells,
a step of removing either 1) cells that express a surface marker, either CD4 or CD8, or 2) cells that express a surface marker αβTCR from the peripheral blood mononuclear cells, and
a step of culturing a cell population obtained by removing cells that express the surface markers in the presence of a bisphosphonate and interleukin-2.

2. The method for producing γδT cells and/or NK cells according to claim 1, wherein the surface markers are CD4 and CD8.

3. The method for producing γδT cells and/or NK cells according to claim 1, wherein peripheral blood mononuclear cells are obtained from peripheral blood of a person infected with HTLV-1.

4. The method for producing γδT cells and/or NK cells according to claim 1, wherein the concentration of the bisphosphonate in the cell culturing is 0.05 to 100 µM, and
the concentration of the interleukin-2 in the cell culturing is 50 to 2000 U/mL.

5. The method for producing γδT cells and/or NK cells according to claim 1, wherein the bisphosphonate is pamidronic acid, alendronic acid, zoledronic acid, risedronic acid, ibandronic acid, incadronic acid or a salt thereof, or a hydrate thereof.

6. A pharmaceutical for treating or preventing cancer or infectious diseases,
the pharmaceutical containing γδT cells produced by a method for producing γδT cells and/or NK cells, the method comprising,
a step of preparing peripheral blood mononuclear cells,
a step of removing either 1) cells that express a surface marker, either CD4 or CD8, or 2) cells that express a surface marker αβTCR from the peripheral blood mononuclear cells, and
a step of culturing peripheral blood mononuclear cells obtained by removing cells that express the surface markers in the presence of a bisphosphonate and interleukin-2.

7. The pharmaceutical according to claim 6, wherein the surface markers are CD4 and CD8.

8. The pharmaceutical according to claim 6, wherein the peripheral blood mononuclear cells are obtained from peripheral blood of a person infected with HTLV-1.

9. The pharmaceutical according to claim 8, which is a pharmaceutical for treating or preventing HTLV-1 associated myelopathy or adult T-cell leukemia.


### SEQUENCE LISTING

### SEQUENCE LISTING

<110> Hokkaido University
   St. Marrianna University
   MEDINET Co., Ltd.
<120> Production method of gamma delta T cell and use thereof
<130> W7387-00
<150> JP 2014-209669
   <151> 2014-10-14
<160> 6
<170> PatentIn version 3.1
<210> 1
   <211> 27
   <212> DNA
   <213> Artificial
<220>
   <223> Primer
<400> 1
   acaaagttaa ccatgcttat tatcagc 27
<210> 2
   <211> 22
   <212> DNA
   <213> Artificial
<220>
   <223> Primer
<400> 2
   acacgtagac tgggtatccg aa 22
<210> 3
   <211> 26
   <212> DNA
   <213> Artificial
<220>
   <223> Probe
<400> 3
   ttcccagggt ttggacagag tcttct 26
<210> 4
   <211> 20
   <212> DNA
   <213> Artificial
<220>
   <223> Primer
<400> 4
   cacactgtgc ccatctacga 20
<210> 5
   <211> 26
   <212> DNA
   <213> Artificial
<220>
   <223> Primer
<400> 5
   ctcagtgagg atcttcatga ggtagt 26
<210> 6
   <211> 26
   <212> DNA
   <213> Artificial
<220>
   <223> Probe
<400> 6
   atgccctccc ccatgccatc ctgcgt 26